# FASCICULE DE BREVET EUROPEEN

(11) **EP 3 407 877 B1**
(45) Date de publication et mention de la délivrance du brevet: **04.03.2020**
(21) Numéro de dépôt: 17706570.3
(22) Date de dépôt: 25.01.2017
(51) Int. Cl.: A61K 31/18, A61K 31/341, A61K 31/357, A61K 31/381, A61K 31/404, A61K 31/4184, A61K 31/473, A61P 17/06, A61P 17/00, A61K 9/00

(54) **INHIBITEURS NLRP3 POUR LE TRAITEMENT DES PATHOLOGIES CUTANÉES INFLAMMATOIRES**
NLRP3-INHIBITOREN ZUR BEHANDLUNG VON ENTZÜNDLICHEN HAUTERKRANKUNGEN
NLRP3 INHIBITORS FOR THE TREATMENT OF INFLAMMATORY SKIN DISORDERS

(30) Priorité: 25.01.2016 FR 1650555
(43) Date de publication de la demande: 05.12.2018
(73) Titulaire: Galderma Research & Development, 06410 Biot (FR)
(72) Inventeur: HACINI-RACHINEL, Feriel, 1807 Blonay (CH)
(74) Mandataire: Nederlandsch Octrooibureau
(86) Numéro de dépôt international: PCT/FR2017/050160
(87) Numéro de publication internationale: WO 2017/129897

(56) Documents cités:
- FR-A1- 2 898 497
- REBECCA C COLL ET AL: "A small-molecule inhibitor of the NLRP3 inflammasome for the treatment of inflammatory diseases", NATURE MEDICINE., 16 février 2015 (2015-02-16), XP055303035, US ISSN: 1078-8956, DOI: 10.1038/nm.3806

## Description

La présente invention concerne le domaine thérapeutique et est plus particulièrement relative à des composés inhibiteurs du récepteur NLRP3 utilisés dans le traitement et/ou la prévention des pathologies cutanées inflammatoires.

### ARRIERE PLAN TECHNOLOGIQUE DE L'INVENTION

Les pathologies cutanées inflammatoires sont des désordres cutanés accompagnés d'une composante inflammatoire. Il existe différents types de pathologies cutanées inflammatoires classées en fonction de leur localisation, de leurs causes et de leurs symptômes. Ce sont des désordres cutanés très communs et leur diagnostic peut parfois être difficile. En effet, le système immunitaire au sein de la peau possède des possibilités limitées de réponse aux stimuli internes et externes et ainsi de très nombreuses pathologies cutanées présentent un profil inflammatoire et ne présentent que très peu de caractéristiques pathologiques spécifiques.

Parmi les pathologies cutanées inflammatoires, la dermatite atopique, maladie inflammatoire chronique qui affecte la peau, se manifeste par des démangeaisons, une sécheresse et des lésions cutanées. La dermatite atopique apparait le plus souvent durant la petite enfance et peut persister ou commencer à l'âge adulte. Cette pathologie est caractérisée par une réponse inappropriée du système immunitaire dirigée contre des antigènes de l'environnement, appelés allergènes. Dans la majorité des cas, les acariens sont à l'origine de la réaction allergique.

L'origine de la dermatite atopique est multifactorielle, avec des interactions complexes entre les facteurs génétiques et l'environnement. En effet, même si dans 30% des cas, la dermatite atopique est associée à une mutation dans le gène de la filaggrine, la forte augmentation de l'incidence de la maladie au cours de ces 30 dernières années dans les pays industrialisés ne peut en aucun cas être expliquée par la génétique, indiquant le rôle fondamental joué par l'environnement. En accord avec cette hypothèse, de nombreuses études épidémiologiques montrent que les personnes élevées dans un milieu rural développent moins de maladies allergiques, dont la dermatite atopique, que les personnes vivant dans des zones industrialisées. Les individus allergiques présentent une prédisposition au développement d'immunoglobulines de type E (IgE), directement responsables des premiers symptômes de l'allergie, ils sont dits « atopiques ».

La dermatite atopique est décrite comme l'étape initiale de la « marche atopique » qui, dans plus de 40% des cas, va conduire à l'apparition d'autres maladies allergiques comme la rhinite allergique ou l'asthme allergique.

Au niveau histologique et immunologique, la dermatite atopique se caractérise par un épaississement de l'épiderme et une infiltration cutanée par des mastocytes, des lymphocytes T auxiliaires, des éosinophiles et des cellules dendritiques.

Il est admis que la phase aigüe de cette maladie est initiée par les lymphocytes T CD4⁺ de type Th2 et Th22. Dans les phases plus chroniques, caractérisées par des lésions lichénifiées de la peau, l'apparition d'autres types lymphocytaires : Th1, et Th17 est observée.

A ce jour, les thérapies disponibles ne sont pas totalement satisfaisantes. En effet, la plupart des traitements offrent un soulagement temporaire et incomplet des symptômes. En outre, l'utilisation à long terme des corticostéroïdes topiques est sujette à des effets secondaires tels que l'atrophie ou l'amincissement de la peau et les inhibiteurs topiques de la calcineurine peuvent causer une irritation locale de la peau ou des brûlures et démangeaisons lorsque le traitement est commencé. Ces effets secondaires peuvent affecter l'observance du patient ou des parents. Enfin, les thérapies systémiques et UV doivent être utilisés avec beaucoup de prudence et doivent être réservés pour les adultes avec des cas graves.

Une autre pathologie cutanée inflammatoire est le psoriasis, qui se caractérise par des plaques rouges, recouvertes de squames blanchâtres qui se détachent de la peau. Ces plaques se localisent notamment au niveau des coudes, genoux, cuir chevelu et bas du dos mais peuvent également toucher d'autres parties du corps. Les cellules de peau se multiplient rapidement et s'accumulent en plaques psoriatiques. La sévérité de cette pathologie peut varier. Cette pathologie semble être multifactorielle et peut également être aggravée par de nombreux facteurs externes tels que le stress, le tabac, l'alcool, l'arrêt de corticostéroïdes etc. Les traitements disponibles à ce jour sont d'une efficacité limitée et peuvent être utilisés seulement sur de courtes périodes. On peut notamment faire référence aux traitements topiques à base de corticostéroïdes, d'analogues de vitamine D, de rétinoïdes, de fluocinonide etc. En revanche ces derniers sont souvent peu efficaces, ne peuvent pas être utilisés sur de longues périodes, et induisent notamment des irritations cutanées ainsi qu'une aggravation de la pathologie lors de l'arrêt du traitement.

Considérant la complexité de la pathophysiologie des pathologies cutanées, telles que la dermatite atopique ou le psoriasis, ainsi que les effets secondaires associés aux traitements disponibles pour ces pathologies cutanées inflammatoires, il existe un besoin de disposer de nouvelles compositions utiles pour le traitement et/ou la prévention de ces pathologies.

Il existe notamment un besoin de disposer d'un traitement présentant une efficacité améliorée, peu d'effets secondaires et qui soit bien toléré notamment chez les jeunes enfants.

### RESUME DE L'INVENTION

Dans ce contexte, le Demandeur propose d'utiliser des composés inhibiteurs du récepteur NLRP3 appartenant à la famille des NOD-like receptor (NLR), en particulier les composés de formule générale (I), pour le traitement et/ou la prévention d'une pathologie cutanée inflammatoire et notamment d'une dermatite atopique ou d'un psoriasis. Un tel inhibiteur est décrit pour traiter des maladies telles que le diabète de type 2, la sclérose en plaque ou la maladie d'Alzheimer (Coll et al., Nature Medicine, 2015, 21 (3), 248-255).

D'autre part, FR2898497 décrit l'utilisation de dérivés d'acide hydroxamique arylsulfonamide, dont la structure diffère de celle des présents composés, pour traiter des pathologies cutanées. L'objet de la présente invention concerne donc un composé de formule générale (I) : 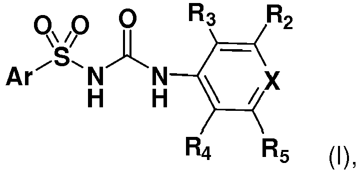 dans laquelle :
- X représente un groupe C-R₁ ou un atome d'azote ;
- R₁ représente un atome d'hydrogène ou un halogène ;
- R₂, R₃, R₄ et R₅ représentent indépendamment un atome d'hydrogène ou un C₁-C₆ alkyle, R₂ et R₃ pris ensemble pouvant former un cyclopentyle avec les atomes de carbone du phényle auxquels ils sont liés, et R₄ et R₅ pris ensemble pouvant former un cyclopentyle avec les atomes de carbone du phényle auxquels ils sont liés ; et
- Ar représente un groupement choisi parmi : 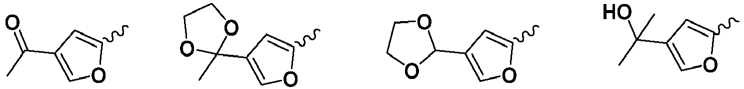 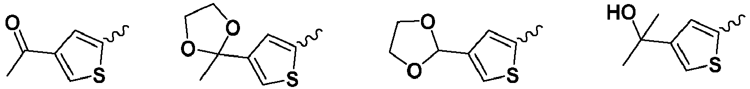 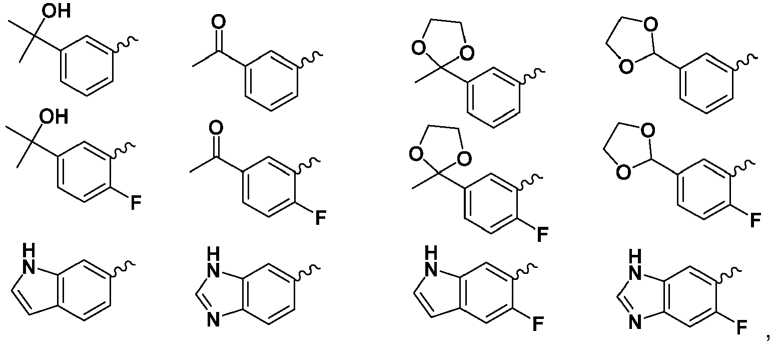
ou un de ses sels, solvates ou hydrates pharmaceutiquement acceptables, pour son utilisation dans le traitement et/ou la prévention des pathologies cutanées inflammatoires.

Dans un mode préféré, les composés tels que définis ici sont utilisés dans le traitement et/ou la prévention d'une dermatite atopique ou d'un psoriasis.

Un autre objet de l'invention est une composition pharmaceutique comprenant un composé tel que défini ici pour son utilisation dans le traitement et/ou la prévention d'une pathologie cutanée inflammatoire, de préférence une dermatite atopique ou un psoriasis.

Est aussi décrite une méthode pour traiter une pathologie cutanée inflammatoire comprenant l'administration d'un composé ou d'une composition pharmaceutique tels que définis ici en une quantité suffisante chez un patient souffrant de ladite pathologie cutanée inflammatoire.

L'invention concerne également l'utilisation d'un composé tel que défini ici pour la fabrication d'un médicament ou d'une composition pharmaceutique destiné à prévenir ou traiter une pathologie cutanée inflammatoire.

Préférentiellement, la composition pharmaceutique utilisée dans la présente invention est destinée à une application topique.

### LEGENDES DES FIGURES

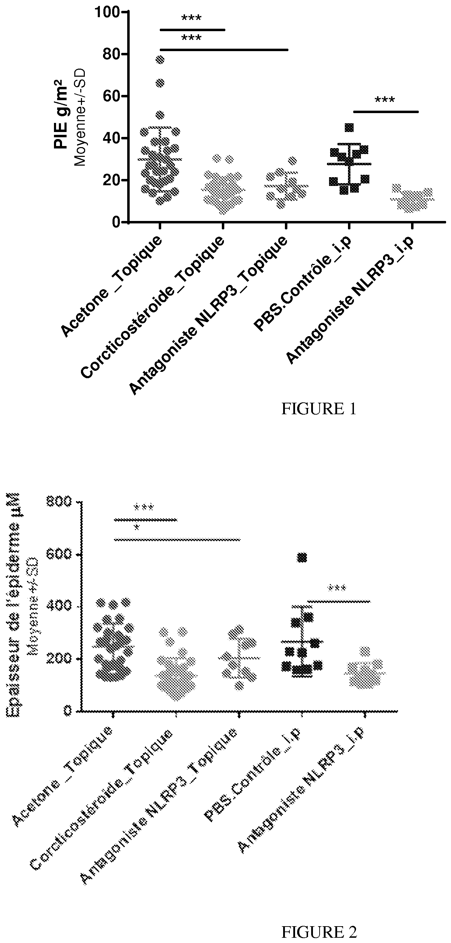

**Figure 1** : Mesure de la perte insensible d'eau au jour 51, après application topique d'acétone (contrôle négatif), du valérate de bétaméthasone à 0,1% (corticostéroïde, témoin positif), et du composé antagoniste NLRP3 (composé n°3, 2% dans un véhicule acétonique), du PBS système (contrôle négatif), et après application systémique du composé antagoniste NLRP3 (composé n°3, 200µl, 2,5 mg / ml une fois par jour).
**Figure 2** : Mesure de l'épaisseur de l'épiderme au jour 51, après application topique d'acétone (contrôle négatif), du valérate de bétaméthasone à 0,1% (corticostéroïde topique, témoin positif), et du composé antagoniste NLRP3 (composé n°3, 2% dans un véhicule acétonique), du PBS système (contrôle négatif), et après application systémique du composé antagoniste NLRP3 (composé n°3, 200µl, 2,5 mg / ml une fois par jour).

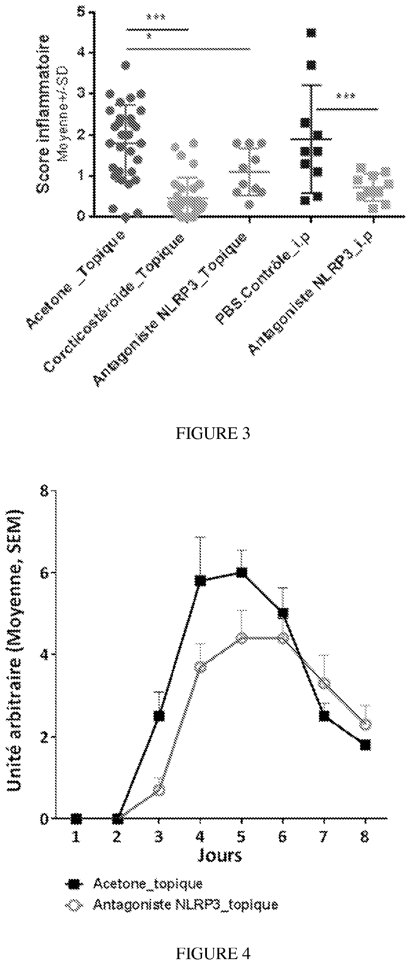

**Figure 3** : Mesure du score inflammatoire au jour 51, après application topique d'acétone (contrôle négatif), du valérate de bétaméthasone à 0,1% (corticostéroïde topique, témoin positif), et du composé antagoniste NLRP3 (composé n°3, 2% dans un véhicule acétonique), du PBS système (contrôle négatif), et après application systémique du composé antagoniste NLRP3 (composé n°3, 200µl, 2,5 mg / ml une fois par jour).
**Figure 4** : Mesure de la cinétique du score clinique du jour 1 à 8 après application topique de 3% du composé antagoniste NLRP3 (composé n°3) dissous dans de l'acétone ou d'acétone seul (contrôle négatif)

### DESCRIPTION DETAILLEE DE L'INVENTION

### Définitions

Le terme « pathologies cutanées inflammatoires » au sens de la présente invention fait référence à tout désordre cutané accompagné d'une composante inflammatoire. Le terme inclut notamment la rosacée, l'acné, l'eczéma, l'eczéma des mains, l'urticaire, l'érythème facial et pudique, le prurit, la dermatite atopique et le psoriasis sous toutes ses formes tel que cutané, muqueux ou unguéal, ou le rhumatisme psoriasique.

Le terme « recepteur NLRP3 » désigne la protéine « NOD-like receptor family, pyrin domain containing 3 », qui est le récepteur le plus étudié de la famille des récepteurs NLR. NLRP3 est impliqué dans la formation d'un complexe appelé « inflammasome » après avoir détecté un ligand. Cet inflammasome clive et ainsi active la caspase-1 qui à son tour peut initier la maturation de l'interleukine 1β (IL-1β) et de l'IL-18 en clivant leurs précurseurs inactifs. L'activation de ces interleukines permet d'activer la voie inflammatoire de mort cellulaire, appelée pyroptose.

Deux étapes sont nécessaires au mécanisme d'activation du NLRP3:
- la première est une étape de reconnaissance de forme induisant la production de la pro-forme d'IL1β et de NLRP3
- la seconde étape consiste à l'assemblage et à l'activation du complexe moléculaire qu'est l'inflammasome, induit par divers ligands.

NLRP3 est impliqué dans la genèse de nombreuses pathologies complexes, notamment en ce qui concerne les désordres métaboliques tels que le diabète de type II, l'athérosclérose ou encore l'obésité. Il semble que NLRP3 soit également impliqué dans des pathologies pulmonaires mais également hépatiques, rénales ou encore liées au vieillissement.

Par composés « inhibiteurs du NLRP3 » au sens de la présente invention, on entend tout composé capable d'inhiber l'activation de l'inflammasome via le récepteur NLRP3 appartenant à la famille des NOD-like receptor (NLR). Plus particulièrement, les composés selon la présente invention sont capables d'inhiber l'activation de l'inflammasome via le NLRP3 grâce à l'inhibition de l'activation de l'IL1β par la voie caspase 1 mais aussi d'inhiber l'activation canonique et non-canonique de l'inflammasome via le récepteur NLRP3. Plus particulièrement, les composés utiles selon la présente invention sont capables d'inhiber spécifiquement le récepteur NLRP3 sans inhiber l'activation d'autres composants de l'inflammasome. De nombreux composés inhibiteurs de NLRP3 sont connus. La société Pfizer a ainsi précédemment développé plusieurs composés du type dérivés de sulfonyle urée substituée, présentant une importante activité inhibitrice de l'activation de l'IL1α et β, notamment grâce à l'inhibition spécifique de NLRP3.

Dans un mode de réalisation, le terme " traitement " ou " traiter " désigne une amélioration, la prophylaxie, ou l'inversion d'une maladie ou d'un trouble, ou au moins d'un symptôme pouvant être discerné de celui-ci. Dans un autre mode de réalisation, "traitement " ou " traiter " désigne une amélioration, la prophylaxie, ou l'inversion d'au moins un paramètre physique mesurable associé à la maladie ou au trouble étant traité, qui n'est pas nécessairement discernable chez ou par le sujet traité. Dans un autre mode de réalisation supplémentaire, " traitement " ou " traiter " désigne l'inhibition ou le ralentissement de la progression d'une maladie ou un trouble, physiquement, par exemple, la stabilisation d'un symptôme discernable, physiologiquement, par exemple, la stabilisation d'un paramètre physique, ou les deux. Dans un autre mode de réalisation, " traitement " ou " traiter " désigne le retard de l'apparition d'une maladie ou trouble.

Dans certains modes de réalisation, les composés sont administrés en tant que mesure préventive. Dans le présent contexte, cette mesure préventive désigne une réduction du risque d'acquisition d'une maladie ou un trouble spécifié mais aussi une réduction, une inhibition ou un ralentissement de l'apparition des symptômes liés à cette maladie. Lorsque la maladie est la dermatite atopique, des symptômes caractéristiques sont par exemple des démangeaisons, une sécheresse et des lésions cutanées. Lorsque la maladie est le psoriasis, des symptômes caractéristiques et sont pour le psoriasis des plaques rouges, recouvertes de squames blanchâtres qui se détachent de la peau.

Au sens de la présente invention, par « sujet » et/ou « patient » on entend tout mammifère, et plus particulièrement les êtres humains, hommes, femmes, enfant. Selon un autre aspect particulier de l'invention, on entend de préférence par « sujet » et/ou « patient », les femmes enceintes, les enfants et les nourrissons.

### Inhibiteurs de NLRP3

Les composés utilisés dans le cadre de la présente invention répondent à la formule générale (I) : 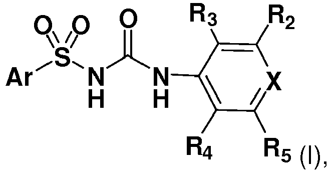 dans laquelle :
- X représente un groupe C-R₁ ou un atome d'azote ;
- R₁ représente un atome d'hydrogène ou un halogène ;
- R₂, R₃, R₄ et R₅ représentent indépendamment un atome d'hydrogène ou un C₁-C₆ alkyle, R₂ et R₃ pris ensemble pouvant former un cyclopentyle avec les atomes de carbone du phényle auxquels ils sont liés, et R₄ et R₅ pris ensemble pouvant former un cyclopentyle avec les atomes de carbone du phényle auxquels ils sont liés ; et
- Ar représente un groupement choisi parmi :

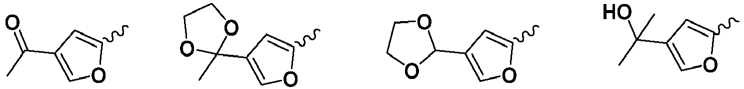

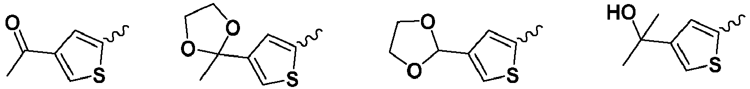

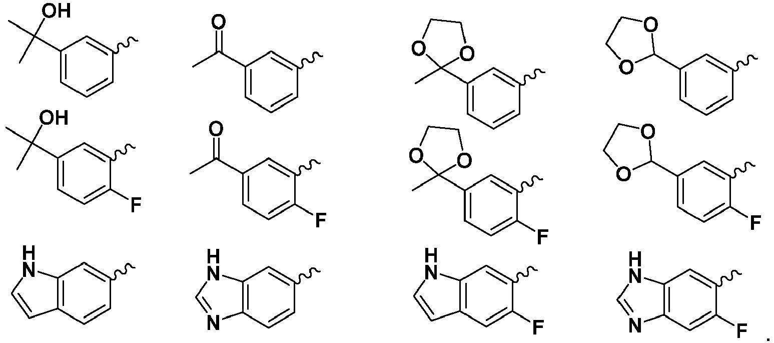

Dans le contexte de la présente invention, les termes ci-dessous ont les significations suivantes :
- Le terme « alkyle » représente un groupe aliphatique saturé, linéaire ou branché, typiquement de 1 à 18 atomes de carbone, de 1 à 10 atomes de carbone, de préférence de 1 à 6 atomes de carbone et encore plus préférentiellement de 1 à 4 atomes de carbone. On peut citer par exemple comme groupe alkyle de 1 à 10 atomes de carbone, les groupes méthyle, éthyle, isopropyle, butyle, tertiobutyle, hexyle, nonyle et dodécyle. Les groupes alkyle de 1 à 6 atomes de carbone (C₁-C₆ alkyle) sont, par exemple, les groupes méthyle, éthyle, isopropyle, butyle, tertiobutyle et hexyle.
- Le terme « cycloalkyle » correspond à un groupe alkyle tel que défini ci-dessus relié par une liaison à ses deux extrémités. On peut citer par exemple le cyclopropyle, le cyclobutyle, le cyclopentyle et le cyclohexyle comme cycloalkyle de 3 à 6 atomes de carbone.
- Le terme « halogène » correspond à un atome de fluor, de chlore, de brome ou d'iode.
- Le terme « Ar » ou « aryle » correspond à un mono- ou bi-cycle ayant 6 à 12 atomes de carbones de formule CₙH₍ₙ₋₂₎. On peut citer par exemple les groupes phényle, biphényle ou naphtyle. Dans le contexte de la présente invention, le terme « Ar » ou « aryle » inclut également les hétéroaryles optionnellement substitués, c'est-à-dire des aryles comprenant au moins un hétéroatome tel qu'un atome d'oxygène, de soufre, et d'azote pouvant être substitués par différents substituants. Par exemple, le terme « Ar » or « aryle » représente un groupement choisi parmi :

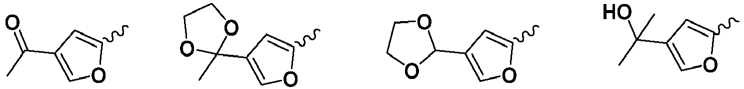

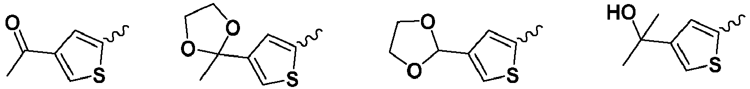

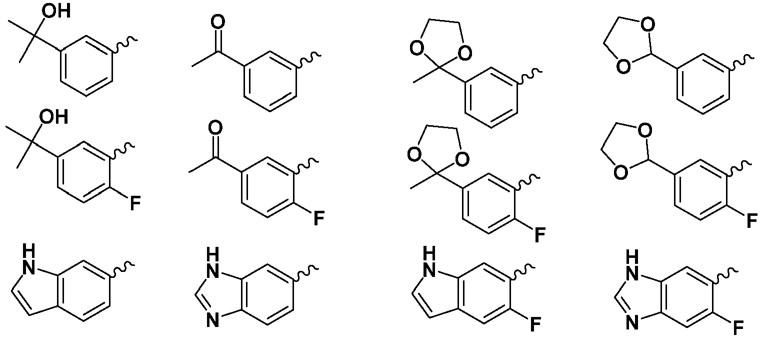

Les composés utilisés dans l'invention comprennent également les sels, les solvates et les hydrates pharmaceutiquement acceptables de ces composés.
- L'expression "sel(s) pharmaceutiquement acceptable(s)" désigne les sels d'un composé d'intérêt qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Une liste de sels pharmaceutiquement acceptables est notamment publiée dans la revue de Berge et al. (J. Pharm. Sci., 1977, 66(1), 1-19).
- Les hydrates correspondent à une combinaison d'un composé de formule (I) avec une ou plusieurs molécules d'eau.
- Les solvates correspondent à une association d'un composé de formule (I) et d'un solvant résultant de la fixation de ce solvant sur les cristaux de composé de formule générale (I) formés en présence de ce solvant.

Dans un premier mode de réalisation particulier, les composés utilisés dans l'invention répondent à formule générale (I) dans laquelle :
- X représente un groupe C-R₁ ou un atome d'azote, R₁ représentant un atome d'hydrogène ou un halogène, de préférence un chlore ou un fluor;
- R₂ et R₃ pris ensemble formant un cyclopentyle avec les atomes de carbone du phényle auxquels ils sont liés ; et
- R₄ et R₅ pris ensemble pouvant former un cyclopentyle avec les atomes de carbone du phényle auxquels ils sont liés.

Les composés selon ce mode de réalisation particulier répondent à la formule générale (II) : 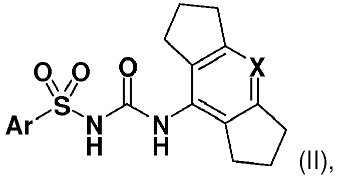 dans laquelle :
- X représente un groupe C-R₁ ou un atome d'azote, R₁ représentant un atome d'hydrogène ou un halogène, de préférence un chlore ou un fluor ; et
- Ar représente un groupement choisi parmi :

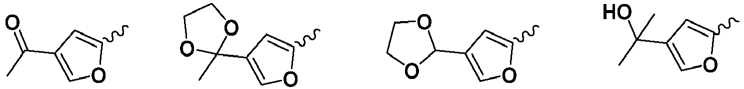

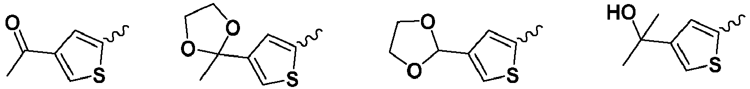

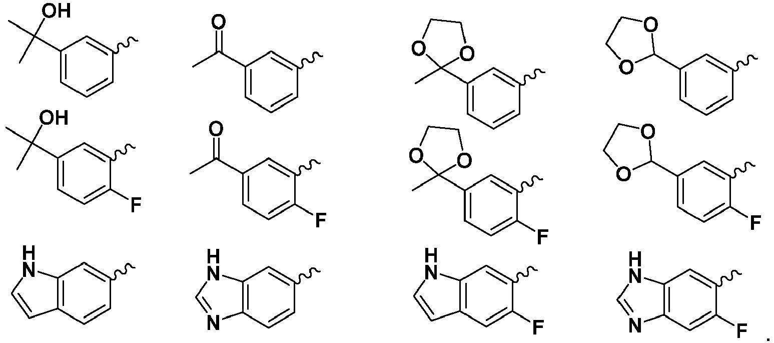

Dans un second mode de réalisation particulier, les composés utilisés dans l'invention répondent à formule générale (I) dans laquelle :
- X représente un groupe C-R₁ ou un atome d'azote, R₁ représentant un atome d'hydrogène ou un halogène, de préférence un chlore ou un fluor ;
- R₂ et R₅ représentent un atome d'hydrogène ; et
- R₃ et R₄ représentent un isopropyle.

Les composés selon ce mode de réalisation particulier répondent à la formule générale (III) : 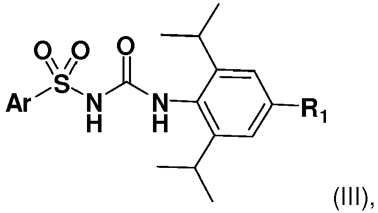 dans laquelle :
- R₁ représente un atome d'hydrogène ou un halogène ; et
- Ar représente un groupement choisi parmi :

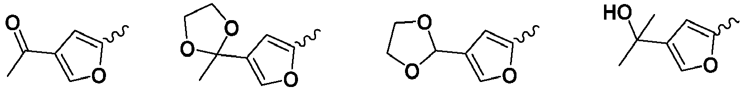

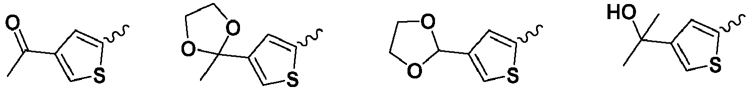

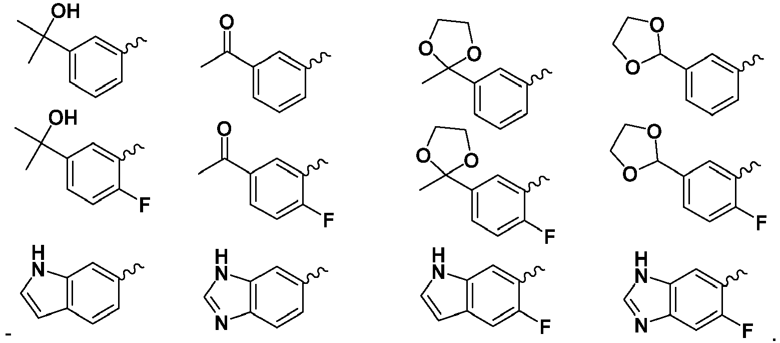

Selon un mode de réalisation préféré, le composé destiné à être utilisé selon la présente invention est choisi parmi les composés suivants :

| **Numéro de composé** | **Nom** | **Structure** |
|---|---|---|
| 1 | N-((4-chloro-2,6-diisopropylphényl)carbamoyl)-3-(2-hydroxypropan-2-yl)benzenesulfonamide | 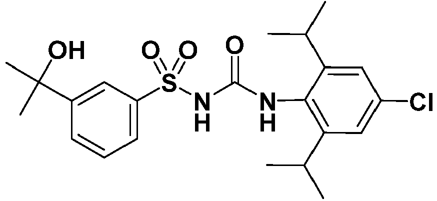 |
| 2 | N-((2,6-diisopropylphényl)carbamoyl) -4-(2-hydroxypropan-2-yl)furan-2-sulfonamide | 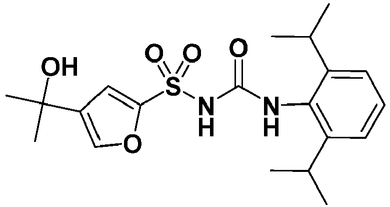 |
| 3 | N-((1,2,3,5,6,7-hexahydro-sindacen-4-yl)carbamoyl)-4-(2-hydroxypropan-2-yl)furan-2-sulfonamide | 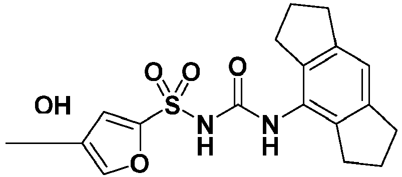 |
| 4 | 4-(1,3-dioxolan-2-yl)-N-((1,2,3,5,6,7-hexahydro-s-indacen-4-yl)carbamoyl)furan-2-sulfonamide | 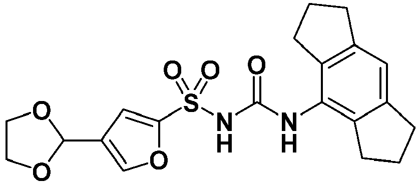 |
| 5 | N-((1,2,3,5,6,7-hexahydrodicyclopenta[b,e]pyridin-8-yl)carbamoyl)-4-(2-hydroxypropan-2-yl)furan-2-sulfonamide | 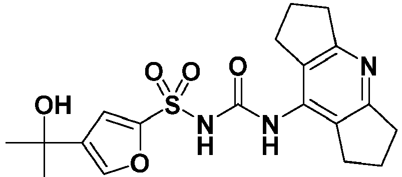 |
| 6 | N-((1,2,3,5,6,7-hexahydro-sindacen-4-yl)carbamoyl)-4-(2-hydroxypropan-2-yl)thiophene-2-sulfonamide | 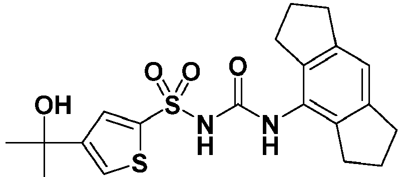 |
| 7 | N-((2,6-diisopropylphényl)carbamoyl)-4-(2-hydroxypropan-2-yl)thiophene-2-sulfonamide | 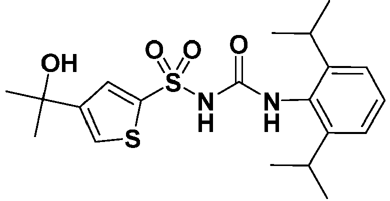 |
| 8 | 4-acetyl-N-((1,2,3,5,6,7-hexahydro-s-indacen-4-yl)carbamoyl)thiophene-2-sulfonamide | 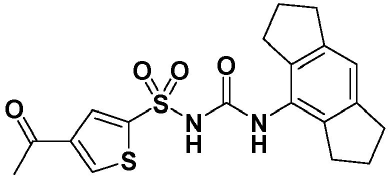 |
| 9 | N-((1,2,3,5,6,7-hexahydro-s-indacen-4-yl)carbamoyl)-1H-benzo[d]imidazole-5-sulfonamide | 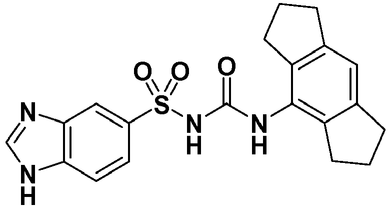 |
| 10 | N-((8-chloro-1,2,3,5,6,7-hexahydro-s-indacen-4-yl)carbamoyl)-4-(2-hydroxypropan-2-yl)furan-2-sulfonamide | 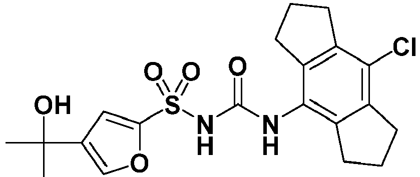 |
| 11 | 4-acetyl-N-((1,2,3,5,6,7-hexahydro-s-indacen-4-yl)carbamoyl)furan-2-sulfonamide | 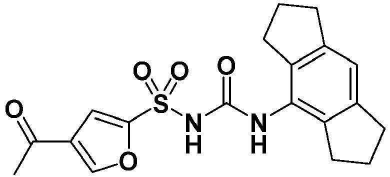 |
| 12 | N-((8-fluoro-1,2,3,5,6,7-hexahydro-s-indacen-4-yl)carbamoyl)-4-(2-hydroxypropan-2-yl)furan-2-sulfonamide | 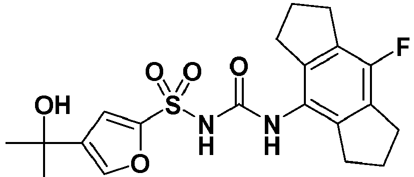 |
| 13 | N-((4-fluoro-2,6-diisopropylphényl)carbamoyl)-3-(2-hydroxypropan-2-yl)benzenesulfonamide | 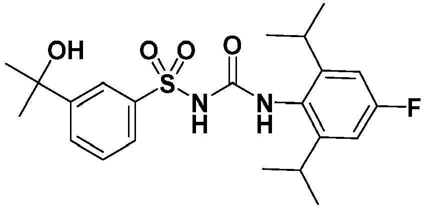 |
| 14 | 6-fluoro-N-((1,2,3,5,6,7-hexahydro-s-indacen-4-yl)carbamoyl)-1H-benzo[d]imidazole-5-sulfonamide | 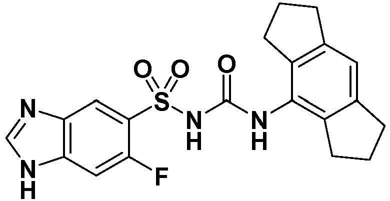 |
| 15 | N-((4-chloro-2,6-diisopropylphényl)carbamoyl)-1H-indole-6-sulfonamide | 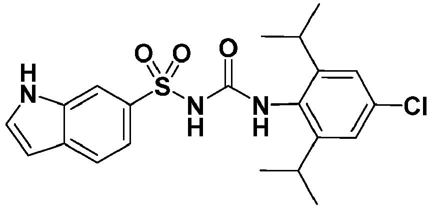 |
| 16 | N-((4-chloro-2,6-diisopropylphényl)carbamoyl) -5-fluoro-1H-indole-6-sulfonamide | 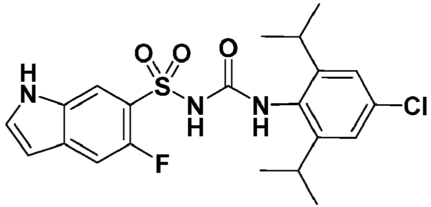 |
| 17 | N-((1,2,3,5,6,7-hexahydro-s-indacen-4-yl)carbamoyl)-1H-indole-6-sulfonamide | 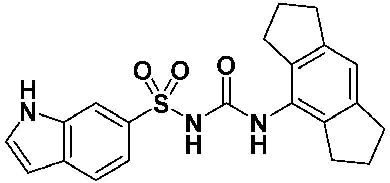 |
| 18 | 5-fluoro-N-((1,2,3,5,6,7-hexahydro-s-indacen-4-yl)carbamoyl)-1H-indole-6-sulfonamide | 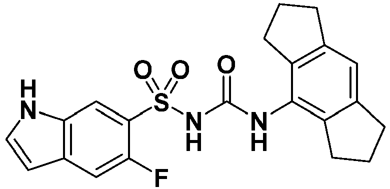 |
| 19 | N-((4-chloro-2,6-diisopropylphényl)carbamoyl) -2-fluoro-5-(2-methyl-1,3-dioxolan-2-yl)benzenesulfonamide | 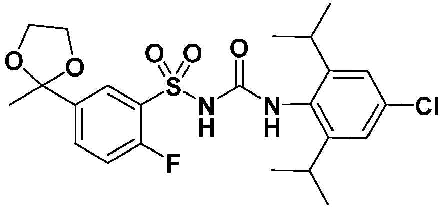 |
| 20 | 2-fluoro-N-((1,2,3,5,6,7-hexahydro-s-indacen-4-yl)carbamoyl)-5-(2-methyl-1,3-dioxolan-2-yl)benzenesulfonamide | 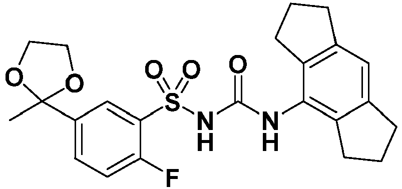 |

Dans un mode de réalisation préféré, le composé utilisé est le N-((1,2,3,5,6,7-hexahydro-s-indacen-4-yl)carbamoyl)-4-(2-hydroxypropan-2-yl)furan-2-sulfonamide.

Ces composés et leurs méthodes de synthèse sont clairement décrits dans la demande de brevet WO98/32733, notamment au sein des exemples. L'homme du métier peut s'y référer pour produire les composés de formule générale (I).

### Compositions pharmaceutiques ou dermatologiques

L'invention concerne également une composition pharmaceutique pouvant comprendre au moins un composé de formule générale (I), un de ses sels, solvates ou hydrates pharmaceutiquement acceptables, destinée à être utilisée dans le traitement et/ou la prévention de la dermatite atopique et/ou du psoriasis.

Dans un mode de réalisation préféré de l'invention le composé de formule générale (I) et la composition pharmaceutique comprenant un composé de formule générale (I), un de ses sels, solvates ou hydrates pharmaceutiquement acceptables sont destinés à une administration topique.

Selon un mode alternatif de l'invention, le composé de formule générale (I) et la composition pharmaceutique comprenant un composé de formule générale (I), un de ses sels, solvates ou hydrates pharmaceutiquement acceptables sont adaptés à une administration orale.

Le composé de formule (I) peut être utilisé comme principe actif Selon un aspect particulier de l'invention, on peut utiliser, pour le traitement d'une pathologie inflammatoire cutanée, et plus particulièrement de la dermatite atopique et du psoriasis, au moins un composé de formule générale (I), ou un de ses sels, solvates ou hydrates pharmaceutiquement acceptables, en association avec un autre principe actif.

La composition pharmaceutique et dermatologique telle que décrite précédemment peut donc contenir des additifs inertes, ou même pharmacodynamiquement actifs, ou des combinaisons de ces additifs, et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des antioxydants, tels que l'α-tocophérol, le butylhydroxyanisole ou le butylhydroxytoluène, la super oxyde dismutase, l'ubiquinol ou certains chélatants de métaux ;
- des agents dépigmentants tels que l'hydroquinone, l'acide azélaïque, l'acide caféïque ou l'acide kojique ;
- des émollients ;
- des agents hydratants comme le glycérol, le PEG 400, la thiamorpholinone, et ses dérivés ou l'urée ;
- des agents anti-inflammatoires non stéroïdiens ;
- des caroténoïdes et, notamment, le β-carotène ;
- des agents anti-psoriatiques tels que l'anthraline et ses dérivés;
- des acides eicosa-5,8,11,14-tétraynoïque et eicosa-5,8,11-triynoïque, leurs esters et amides ;
- des rétinoïdes, c'est à dire des ligands des récepteurs RAR ou RXR, naturels ou synthétiques ;
- des ligands des récepteurs VDR ;
- des corticostéroïdes ou des œstrogènes ;
- des α-hydroxy acides et des α-céto acides ou leurs dérivés, tels que les acides lactique, malique, citrique, glycolique, mandélique, tartrique, glycérique, ascorbique, ainsi que leurs sels, amides ou esters, ou des β-hydroxy acides ou leurs dérivés, tels que l'acide salicylique ainsi que ses sels, amides ou esters ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les récepteurs solubles, les cytokines ou les facteurs de croissance...).

L'homme du métier sait choisir le ou les éventuels composés à ajouter à ces compositions de telle manière que l'effet désiré ne soit pas, ou substantiellement pas altéré par l'addition envisagée.

Une telle composition peut être destinée, et donc adaptée, à une administration par voie orale, topique, entérale, parentérale, oculaire, sublinguale, inhalée, sous-cutanée, intramusculaire, intraveineuse, transdermique, locale ou rectale. Le composé de formule générale (I), éventuellement sous la forme de sel, solvate et/ou hydrate, pharmaceutiquement acceptable, seul ou en association avec un autre principe actif, peut être administré sous une forme unitaire d'administration, en mélange avec des supports ou excipients pharmaceutiques classiques, aux animaux et aux êtres humains.

De préférence, la composition pharmaceutique est conditionnée sous une forme convenant à une administration par voie topique.

La composition pharmaceutique comprenant au moins un composé de formule générale (I) destinée à son utilisation selon la présente invention est de préférence formulée en composition dermatologique.
La composition utile selon l'invention comprend un support physiologiquement acceptable ou au moins un excipient pharmaceutiquement acceptable, choisi selon la forme pharmaceutique, notamment dermatologique, souhaitée et le mode d'administration choisi. Par support physiologiquement acceptable et excipient pharmaceutiquement acceptable, on entend respectivement, pour une application topique, un support et un excipient compatibles avec la peau, les muqueuses et les phanères.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de syndets, de solutions, de gels, de sprays, de mousses, de suspensions, de lotions, de sticks, de shampoings, ou de bases lavantes. Elle peut également se présenter sous forme de suspensions de microsphères ou nanosphères ou vésicules lipidiques ou polymériques ou de patches polymériques et d'hydrogels permettant une libération contrôlée. Cette composition par voie topique peut se présenter sous forme anhydre, sous forme aqueuse ou sous la forme d'une émulsion.

Le composés de formule générale (I), ou un de ses sels, solvates ou hydrates, lorsqu'il est administré par voie topique peut être notamment utilisé à une concentration généralement comprise entre 0,001 et 10% en poids, de préférence entre 0,01 et 5% en poids, par rapport au poids total de la composition.

### Applications thérapeutiques

Selon la présente invention, les composés décrits ici sont utilisés pour le traitement et/ou la prévention d'une pathologie cutanée inflammatoire, notamment d'une dermatite atopique et/ou un psoriasis, de préférence par voie topique.

Les composés destinés à être utilisés selon la présente invention en application topique présentent une bonne pénétration cutanée, et une bonne tolérance et une faible toxicité avec peu ou pas d'effets secondaires.

Selon un aspect préféré de l'invention les sujets préférés pour le traitement et/ou la prévention de la dermatite atopique et/ou du psoriasis sont les femmes enceintes, les enfants et les nourrissons.

La composition utile selon l'invention comprend au moins un composé de formule générale (I), ou un de ses sels, solvates ou hydrates pharmaceutiquement acceptables, en quantité suffisante pour obtenir l'effet prophylactique ou thérapeutique souhaité. La posologie utile varie selon l'âge, le sexe et le poids du patient.

Le composé de formule générale (I), ou un de ses sels, solvates ou hydrates peut être de préférence, administré à raison de 0,01 à 100 mg/kg et par jour, avantageusement de 0,01 à 50 mg/kg et par jour. Il est également possible d'administrer de telles doses, en 2 à 4 administrations quotidiennes. Bien que ces dosages soient des exemples de situations moyennes, il peut y avoir des cas particuliers où des dosages plus élevés ou plus faibles sont appropriés, de tels dosages appartiennent également à l'invention.

### EXEMPLES

**Exemple 1:** Protocole d'essai d'un antagoniste NLRP3 (N-((1,2,3,5,6,7-hexahydro-s-indacen-4-yl)carbamoyl)-4-(2-hydroxypropan-2-yl)furan-2-sulfonamide, composé 3, aussi appelé MCC950) sur un modèle de souris dermatite atopique.

Des souris Balb/c femelles ont été traitées avec l'allergène *Dermatophagoïdes pteronyssinus* (DERP) dans 3 cycles de sensibilisation épicutanée (par patch) sur la peau abdominale avec 2 patchs appliqués deux fois par semaine suivis de 2 semaines de repos pendant 7 semaines. Des traitements topiques avec un antagoniste NLRP3 (composé 3, 2% dans un véhicule acétonique) ou un corticostéroïde (valérate de bétaméthasone à 0,1%, utilisé comme témoin) ont été réalisés 3 fois au cours de la troisième et dernière semaine de sensibilisation (aux jours 44, 47 et 50).

Une réaction inflammatoire a été induite par une sensibilisation épicutanée avec des patchs imprégnés de 100 µg d'allergène dans une solution saline stérile (Dermatophagoïdes pteronyssinus ou DERP) ou avec un véhicule appliqué sur la peau abdominale 24 heures après le rasage et laissé pendant trois périodes de 1 semaine (avec un changement de patch en milieu de semaine), avec un intervalle de 2 semaines entre les applications.
Pour la voie systémique, l'antagoniste de NLRP3 a été administré par voie intraveineuse, 200 µl, 2,5 mg / ml une fois par jour, pendant la dernière série de sensibilisation épicutanée avec l'allergène (J44 à J50).
Pour l'administration topique, l'antagoniste d'essai (composé 3) a été formulé à 2% dans un véhicule acétonique, appliqué sur la peau sensibilisée 2 heures avant l'installation des patchs DERP. Les traitements topiques avec un antagoniste de NLRP3 ou un corticostéroïde (utilisé comme témoin) ont été réalisés 3 fois au cours de la troisième et dernière semaine de sensibilisation (aux jours 44, 47 et 50). Au moment du retrait du dernier patch (jour 51), les échantillons de peau et de sang ont été receuillis.

### - Mesures

### • Perte insensible en eau (PIE) après traitement par un antagoniste NLRP3

La PIE est mesurée à l'aide d'un Tewameter sur un animal la veille du jour 51. La sonde Tewameter® mesure le gradient de densité de l'évaporation d'eau de la peau indirectement par deux capteurs (température et humidité relative) à l'intérieur d'un cylindre creux. Un microprocesseur analyse les valeurs et exprime la vitesse d'évaporation en g / h / m2.

### • Epaisseur de l'épiderme après traitement par un antagoniste NLRP3

L'épaisseur de l'épiderme est mesurée par des analyses morphométriques histologiques sur des coupes de 6µm d'épaisseur, colorées par hématoxyline éosine (HE), provenant d'échantillons de peau fixés préalablement au sérum physiologique.

### • Score inflammatoire après traitement par un antagoniste NLRP3

Le score inflammatoire est défini visuellement en se fondant sur des échelles prédéfinies de sécheresse, d'éruption cutanée, de calvitie, d'excoriation et de lichénification. La moyenne des cinq scores donnés à une souris individuelle un jour donné indique le score clinique donné le jour spécifique.

### - Résultats

- Les résultats sont montrés sur les figures 1 à 3. L'antagoniste topique et systémique de NLRP3 ont permis de réduire tous les paramètres étudiés. La PIE qui est un signe clinique de dysfonctionnement de la barrière cutanée, a été significativement diminuée (-60% pour la voie systémique et -42% pour la voie topique). Un autre paramètre du défaut de barrière cutanée, l'acanthose de l'épiderme, a été partiellement restauré avec l'antagoniste de NLRP3 (-38% pour la voie systémique et -24% pour la voie topique, sur l'épaisseur de l'épiderme). Les paramètres inflammatoires ont également été réduits tels que les scores inflammatoires (-62% pour la voie systémique et -39% pour la voie topique). L'efficacité de l'antagoniste de NLRP3 testé est assez proche du traitement aux corticostéroïdes qui sont les traitements de référence de la dermatite atopique.

**Exemple 2:** Protocole d'essai d'un antagoniste NLRP3 (N-((1,2,3,5,6,7-hexahydro-s-indacen-4-yl)carbamoyl)-4-(2-hydroxypropan-2-yl)furan-2-sulfonamide, composé 3, aussi appelé MCC950) sur un modèle de souris psoriasiforme.

Le protocole de traitement consiste en une application topique quotidienne d'Aldara® (3,18 mg d'imiquimod) pendant 7 jours sur la peau rasée des souris Balb / c. L'antagoniste NLRP3 a été dissous dans de l'acétone à 3% et a été appliqué une fois par jour 2h avant le traitement par Aldara®. Le score clinique est un classement global résultant de la somme des scores d'érythème, d'œdème et de desquamation inspirés de l'évaluation globale du médecin du système Lattice, connue sous le nom de LS PGA (J Am Acad Dermatol. 2004 Oct;51(4):563-9).

L'application topique répétée d'imiquimod (IMQ) sur la peau des souris a permis de mettre en évidence une réduction de l'inflammation cutanée ressemblant au phénotype du psoriasis humain (PLoS One, 2011). En effet, l'imiquimod induit des lésions de peau écailleuse enflammées ressemblant à la plaque de type psoriasis. Ces lésions sont associées à une prolifération épidermique accrue, une différenciation anormale, une accumulation épidermique de neutrophiles dans des micro-traitements, une néoangiogenèse et des infiltrats constitués de cellules T CD4 +, de cellules dendritiques CD11c (+) et de cellules dendritiques plasmacytoïdes. L'imiquimod induit une expression épidermique d'IL-23, IL-17A et IL-17F, ainsi qu'une augmentation des cellules Th17 spléniques

Les souris ont reçu une dose quotidienne de 63,5 mg (14 µl) de crème d'imiquimod disponible dans le commerce (5%) (Aldara®, 3M Pharmaceuticals) sur la peau rasée du dos de souris pendant 7 jours consécutifs.

### - Résultats

Dans ce modèle, l'administration topique de l'antagoniste NLRP3 a permis de réduire l'inflammation cutanée induite par l'imiquimod (-36% au jour 4 et -27% au jour 5 sur la base du score clinique, figure 4).

## Revendications

1. Composé de formule générale (I) : 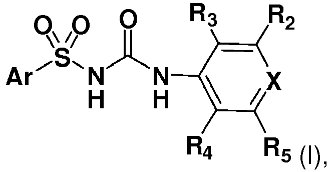 dans laquelle :
- X représente un groupe C-R₁ ou un atome d'azote ;
- R₁ représente un atome d'hydrogène ou un halogène ;
- R₂, R₃, R₄ et R₅ représentent indépendamment un atome d'hydrogène ou un C₁-C₆ alkyle, R₂ et R₃ pris ensemble pouvant former un cyclopentyle avec les atomes de carbone du phényle auxquels ils sont liés, et R₄ et R₅ pris ensemble pouvant former un cyclopentyle avec les atomes de carbone du phényle auxquels ils sont liés ; et
- Ar représente un groupement choisi parmi : 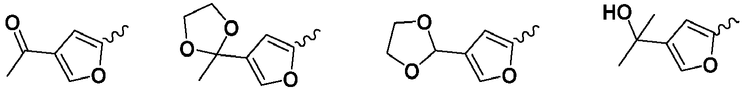 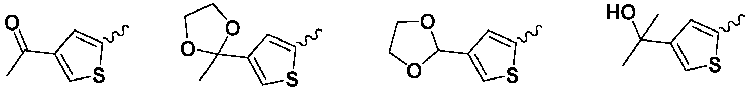 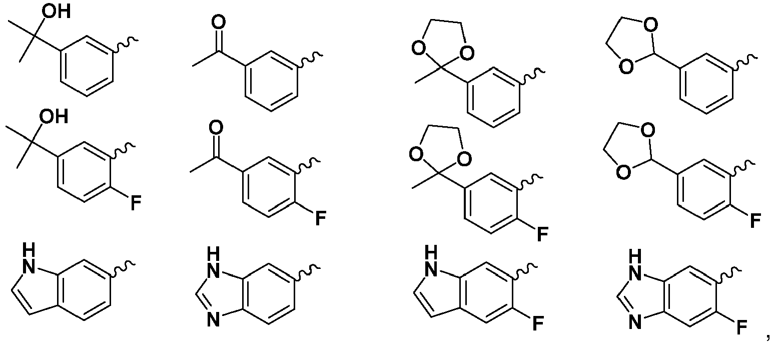 ou un de ses sels, solvates ou hydrates pharmaceutiquement acceptables, pour son utilisation dans le traitement et/ou la prévention d'une pathologie cutanée inflammatoire.

2. Composé pour son utilisation selon la revendication 1, dans lequel :
- X représente un groupe C-R₁ ou un atome d'azote, R₁ étant tel que défini dans la revendication 1 ;
- R₂ et R₃ pris ensemble formant un cyclopentyle avec les atomes de carbone du phényle auxquels ils sont liés ; et
- R₄ et R₅ pris ensemble pouvant former un cyclopentyle avec les atomes de carbone du phényle auxquels ils sont liés.

3. Composé pour son utilisation selon la revendication 1, dans laquelle :
- X représente un groupe C-R₁ ou un atome d'azote, R₁ étant tel que défini dans la revendication 1 ;
- R₂ et R₅ représentent un atome d'hydrogène ; et
- R₃ et R₄ représentent un isopropyle.

4. Composé pour son utilisation selon l'une quelconque des revendications 1 à 3, dans lequel l'halogène est un chlore ou un fluor.

5. Composé pour son utilisation selon l'une quelconque des revendications 1 à 4, **caractérisé en ce qu'**il est choisi parmi les composés suivants :
- Composé 1 : N-((4-chloro-2,6-diisopropylphényl)carbamoyl)-3-(2-hydroxypropan-2-yl)benzenesulfonamide ;
- Composé 2 : N-((2,6-diisopropylphényl)carbamoyl)-4-(2-hydroxypropan-2-yl)furan-2-sulfonamide ;
- Composé 3 : N-((1,2,3,5,6,7-hexahydro-s-indacen-4-yl)carbamoyl)-4-(2-hydroxypropan-2-yl)furan-2-sulfonamide ;
- Composé 4 : 4-(1,3-dioxolan-2-yl)-N-((1,2,3,5,6,7-hexahydro-s-indacen-4-yl)carbamoyl)furan-2-sulfonamide ;
- Composé 5 : N-((1,2,3,5,6,7-hexahydrodicyclopenta[b,e]pyridin-8-yl)carbamoyl)-4-(2-hydroxypropan-2-yl)furan-2-sulfonamide ;
- Composé 6 : N-((1,2,3,5,6,7-hexahydro-s-indacen-4-yl)carbamoyl)-4-(2-hydroxypropan-2-yl)thiophene-2-sulfonamide ;
- Composé 7 : N-((2,6-diisopropylphenyl)carbamoyl)-4-(2-hydroxypropan-2-yl)thiophene-2-sulfonamide ;
- Composé 8 : 4-acétyl-N-((1,2,3,5,6,7-hexahydro-s-indacen-4-yl)carbamoyl)thiophene-2-sulfonamide ;
- Composé 9 : N-((1,2,3,5,6,7-hexahydro-s-indacen-4-yl)carbamoyl)-1H-benzo[d]imidazole-5-sulfonamide ;
- Composé 10 : N-((8-chloro-1,2,3,5,6,7-hexahydro-s-indacen-4-yl)carbamoyl)-4-(2-hydroxypropan-2-yl)furan-2-sulfonamide ;
- Composé 11 : 4-acétyl-N-((1,2,3,5,6,7-hexahydro-s-indacen-4-yl)carbamoyl)furan-2-sulfonamide ;
- Composé 12 : N-((8-fluoro-1,2,3,5,6,7-hexahydro-s-indacen-4-yl)carbamoyl)-4-(2-hydroxypropan-2-yl)furan-2-sulfonamide ;
- Composé 13 : N-((4-fluoro-2,6-diisopropylphényl)carbamoyl)-3-(2-hydroxypropan-2-yl)benzenesulfonamide ;
- Composé 14 : 6-fluoro-N-((1,2,3,5,6,7-hexahydro-s-indacen-4-yl)carbamoyl)-1H-benzo[d]imidazole-5-sulfonamide ;
- Composé 15 : N-((4-chloro-2,6-diisopropylphényl)carbamoyl)-1H-indole-6-sulfonamide ;
- Composé 16 : N-((4-chloro-2,6-diisopropylphényl)carbamoyl)-5-fluoro-1H-indole-6-sulfonamide ;
- Composé 17 : N-((1,2,3,5,6,7-hexahydro-s-indacen-4-yl)carbamoyl)-1H-indole-6-sulfonamide ;
- Composé 18 : 5-fluoro-N-((1,2,3,5,6,7-hexahydro-s-indacen-4-yl)carbamoyl)-1H-indole-6-sulfonamide ;
- Composé 19 : N-((4-chloro-2,6-diisopropylphényl)carbamoyl)-2-fluoro-5-(2-methyl-1,3-dioxolan-2-yl)benzenesulfonamide ; et
- Composé 20 : 2-fluoro-N-((1,2,3,5,6,7-hexahydro-s-indacen-4-yl)carbamoyl)-5-(2-methyl-1,3-dioxolan-2-yl)benzenesulfonamide.

6. Composé pour son utilisation selon l'une quelconques des revendications 1, 2, 4, ou 5, **caractérisé en ce que** le composé est le N-((1,2,3,5,6,7-hexahydro-s-indacen-4-yl)carbamoyl)-4-(2-hydroxypropan-2-yl)furan-2-sulfonamide.

7. Composé pour son utilisation selon l'une quelconque des revendications 1 à 6, dans le traitement et/ou la prévention d'une dermatite atopique.

8. Composé pour son utilisation selon l'une quelconque des revendications 1 à 6, dans le traitement et/ou la prévention d'un psoriasis.

9. Composition pharmaceutique comprenant un composé tel que défini selon l'une quelconque des revendications 1 à 6, pour son utilisation dans le traitement et/ou la prévention d'une pathologie cutanée inflammatoire, de préférence une dermatite atopique ou un psoriasis.

10. Composition pharmaceutique pour une utilisation selon la revendication 9, par application topique.

## Patentansprüche

1. Verbindung der allgemeinen Formel (I): 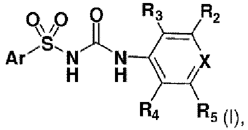 wobei:
- X für eine C-R₁ Gruppe oder ein Stickstoffatom steht;
- R₁ für ein Wasserstoff- oder ein Halogenatom steht;
- R₂, R₃, R₄ und R₅ unabhängig voneinander für ein Wasserstoffatom oder ein C₁-C₆-Alkyl stehen, wobei R₂ und R₃ zusammen mit den Kohlenstoffatomen des Phenyls, an die sie gebunden sind, ein Cyclopentyl bilden können, und R₄ und R₅ zusammen mit den Kohlenstoffatomen des Phenyls, an die sie gebunden sind, ein Cyclopentyl bilden können; und
- Ar für eine Gruppe steht, die aus Folgendem ausgewählt ist: 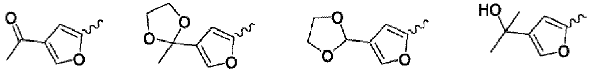 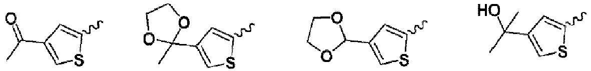 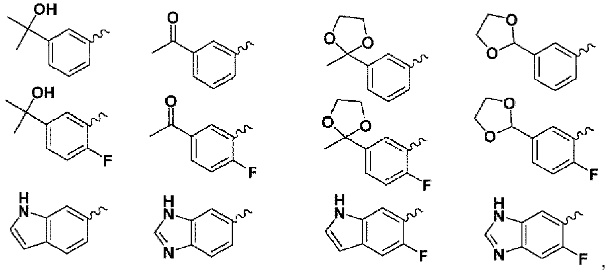 oder ein pharmazeutisch verträgliches Salz, Solvat oder Hydrat davon zur Verwendung bei der Behandlung und / oder Prävention einer entzündlichen Hauterkrankung.

2. Verbindung zur Verwendung nach Anspruch 1, wobei,
- X für eine C-R₁-Gruppe oder ein Stickstoffatom steht, R₁ wie in Anspruch 1 definiert ist;
- R₂ und R₃ zusammen mit den Kohlenstoffatomen des Phenyls, an die sie gebunden sind, ein Cyclopentyl bilden; und
- R₄ und R₅ zusammen mit den Kohlenstoffatomen des Phenyls, an die sie gebunden sind, ein Cyclopentyl bilden können.

3. Verbindung zur Verwendung nach Anspruch 1, wobei:
- X für eine C-R₁-Gruppe oder ein Stickstoffatom steht, R₁ wie in Anspruch 1 definiert ist;
- R₂ und R₅ für ein Wasserstoffatom stehen; und
- R₃ und R₄ für Isopropyl stehen.

4. Verbindung zur Verwendung nach einem der Ansprüche 1 bis 3, wobei das Halogen Chlor oder Fluor ist.

5. Verbindung zur Verwendung nach einem der Ansprüche 1 bis 4, **dadurch gekennzeichnet, dass** sie aus den folgenden Verbindungen ausgewählt ist:
- Verbindung 1: N-((4-chlor-2,6-diisopropylphenyl)carbamoyl)-3-(2-hydroxypropan-2-yl)benzensulfonamid;
- Verbindung 2: N-((2,6-diisopropylphenyl)carbamoyl)-4-(2-hydroxypropan-2-yl)furan-2-sulfonamid;
- Verbindung 3: N-((1,2,3,5,6,7-hexahydro-s-indacen-4-yl)carbamoyl)-4-(2-hydroxypropan-2-yl)furan-2-sulfonamid;
- Verbindung 4: 4-(1,3-dioxolan-2-yl)-N-((1,2,3,5,6,7-hexahydro-s-indacen-4-yl)carbamoyl)furan-2-sulfonamid;
- Verbindung 5: N-((1,2,3,5,6,7-hexahydrodicyclopenta[b,e]pyridin-8-yl)carbamoyl)-4-(2-hydroxypropan-2-yl)furan-2-sulfonamid;
- Verbindung 6: N-((1,2,3,5,6,7-hexahydro-s-indacen-4-yl)carbamoyl)-4-(2-hydroxypropan-2-yl)thiophen-2-sulfonamid;
- Verbindung 7: N-((2,6-diisopropylphenyl)carbamoyl)-4-(2-hydroxypropan-2-yl)thiophen-2-sulfonamid;
- Verbindung 8: 4-acetyl-N-((1,2,3,5,6,7-hexahydro-s-indacen-4-yl)carbamoyl)thiophen-2-sulfonamid;
- Verbindung 9: N-((1,2,3,5,6,7-hexahydro-s-indacen-4-yl)carbamoyl)-1H-benzo[d]imidazol-5-sulfonamid;
- Verbindung 10: N-((8-chlor-1,2,3,5,6,7-hexahydro-s-indacen-4-yl)carbamoyl)-4-(2-hydroxypropan-2-yl)furan-2-sulfonamid;
- Verbindung 11: 4-acetyl-N-((1,2,3,5,6,7-hexahydro-s-indacen-4-yl)carbamoyl)furan-2-sulfonamid;
- Verbindung 12: N-((8-fluor-1,2,3,5,6,7-hexahydro-s-indacen-4-yl)carbamoyl)-4-(2-hydroxypropan-2-yl)furan-2-sulfonamid;
- Verbindung 13: N-((4-fluor-2,6-diisopropylphenyl)carbamoyl)-3-(2-hydroxypropan-2-yl)benzensulfonamid;
- Verbindung 14: 6-fluor-N-((1,2,3,5,6,7-hexahydro-s-indacen-4-yl)carbamoyl)-1H-benzo[d]imidazol-5-sulfonamid;
- Verbindung 15: N-((4-chlor-2,6-diisopropylphenyl)carbamoyl)-1H-indole-6-sulfonamid;
- Verbindung 16: N-((4-chlor-2,6-diisopropylphenyl)carbamoyl)-5-fluor-1H-indole-6-sulfonamid;
- Verbindung 17: N-((1,2,3,5,6,7-hexahydro-s-indacen-4-yl)carbamoyl)-1H-indole-6-sulfonamid;
- Verbindung 18: 5-fluor-N-((1,2,3,5,6,7-hexahydro-s-indacen-4-yl)carbamoyl)-1H-indole-6-sulfonamid;
- Verbindung 19: N-((4-chlor-2,6-diisopropylphenyl)carbamoyl)-2-fluor-5-(2-methyl-1,3-dioxolan-2-yl)benzensulfonamid; und
- Verbindung 20: 2-fluor-N-((1,2,3,5,6,7-hexahydro-s-indacen-4-yl)carbamoyl)-5-(2-methyl-1,3-dioxolan-2-yl)benzensulfonamid.

6. Verbindung zur Verwendung nach einem der Ansprüche 1, 2, 4, oder 5, **dadurch gekennzeichnet, dass** die Verbindung N-((1,2,3,5,6,7-hexahydro-s-indacen-4-yl)carbamoyl)-4-(2-hydroxypropan-2-yl)furan-2-sulfonamid ist.

7. Verbindung zur Verwendung nach einem der Ansprüche 1 bis 6 bei der Behandlung und / oder Prävention von atopischer Dermatitis.

8. Verbindung zur Verwendung nach einem der Ansprüche 1 bis 6 bei der Behandlung und / oder Prävention von Psoriasis.

9. Pharmazeutische Zusammensetzung, umfassend eine Verbindung nach einem der Ansprüche 1 bis 6 zur Verwendung bei der Behandlung und / oder Prävention einer entzündlichen Hauterkrankung, vorzugsweise atopischer Dermatitis oder Psoriasis.

10. Pharmazeutische Zusammensetzung zur Verwendung nach Anspruch 9, durch topische Anwendung.

## Claims

1. Compound of general formula (I): 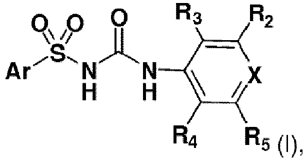 wherein:
- X represents a C-R₁ group or a nitrogen atom;
- R₁ represents a hydrogen or a halogen atom;
- R₂, R₃, R₄ and R₅ independently represent a hydrogen atom or a C₁-C₆ alkyl, R₂ and R₃ together being able to form a cyclopentyl with the carbon atoms from the phenyl to which they are attached, and R₄ and R₅ together being able to form a cyclopentyl with the carbon atoms from the phenyl to which they are attached; and
- Ar represents a group selected from: 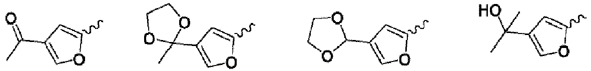 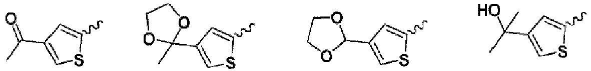 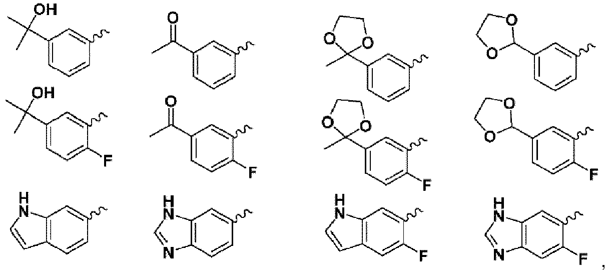
or a pharmaceutically acceptable salt, solvate or hydrate thereof for use in the treatment and/or prevention of an inflammatory skin disorder.

2. Compound for use according to claim 1, wherein:
- X represents a C-R₁ group or a nitrogen atom, R₁ being as defined in claim 1;
- R₂ and R₃ together forming a cyclopentyl with the carbon atoms from the phenyl to which they are attached; and
- R₄ and R₅ together being able to form a cyclopentyl with the carbon atoms from the phenyl to which they are attached.

3. Compound for use according to claim 1, wherein:
- X represents a C-R₁ group or a nitrogen atom, R₁ being as defined in claim 1;
- R₂ and R₅ represent a hydrogen atom; and
- R₃ and R₄ represent an isopropyl.

4. Compound for use according to any of claims 1 to 3, wherein the halogen is chlorine or fluorine.

5. Compound for use according to any one of claims 1 to 4, **characterized in that** it is selected from the following compounds:
- Compound 1: N-((4-chloro-2,6-diisopropylphenyl)carbamoyl)-3-(2-hydroxypropan-2-yl)benzenesulfonamide;
- Compound 2: N-((2,6-diisopropylphenyl)carbamoyl)-4-(2-hydroxypropan-2-yl)furan-2-sulfonamide;
- Compound 3: N-((1,2,3,5,6,7-hexahydro-s-indacen-4-yl)carbamoyl)-4-(2-hydroxypropan-2-yl)furan-2-sulfonamide;
- Compound 4: 4-(1,3-dioxolan-2-yl)-N-((1,2,3,5,6,7-hexahydro-s-indacen-4-yl)carbamoyl)furan-2-sulfonamide;
- Compound 5: N-((1,2,3,5,6,7-hexahydrodicyclopenta[b,e]pyridin-8-yl)carbamoyl)-4-(2-hydroxypropan-2-yl)furan-2-sulfonamide;
- Compound 6: N-((1,2,3,5,6,7-hexahydro-s-indacen-4-yl)carbamoyl)-4-(2-hydroxypropan-2-yl)thiophene-2-sulfonamide;
- Compound 7: N-((2,6-diisopropylphenyl)carbamoyl)-4-(2-hydroxypropan-2-yl)thiophene-2-sulfonamide;
- Compound 8: 4-acetyl-N-((1,2,3,5,6,7-hexahydro-s-indacen-4-yl)carbamoyl)thiophene-2-sulfonamide;
- Compound 9: N-((1,2,3,5,6,7-hexahydro-s-indacen-4-yl)carbamoyl)-1H-benzo[d]imidazole-5-sulfonamide;
- Compound 10: N-((8-chloro-1,2,3,5,6,7-hexahydro-s-indacen-4-yl)carbamoyl)-4-(2-hydroxypropan-2-yl)furan-2-sulfonamide;
- Compound 11: 4-acetyl-N-((1,2,3,5,6,7-hexahydro-s-indacen-4-yl)carbamoyl)furan-2-sulfonamide;
- Compound 12: N-((8-fluoro-1,2,3,5,6,7-hexahydro-s-indacen-4-yl)carbamoyl)-4-(2-hydroxypropan-2-yl)furan-2-sulfonamide;
- Compound 13: N-((4-fluoro-2,6-diisopropylphenyl)carbamoyl)-3-(2-hydroxypropan-2-yl)benzenesulfonamide;
- Compound 14: 6-fluoro-N-((1,2,3,5,6,7-hexahydro-s-indacen-4-yl)carbamoyl)-1H-benzo[d]imidazole-5-sulfonamide;
- Compound 15: N-((4-chloro-2,6-diisopropylphenyl)carbamoyl)-1H-indole-6-sulfonamide;
- Compound 16: N-((4-chloro-2,6-diisopropylphenyl)carbamoyl)-5-fluoro-1H-indole-6-sulfonamide;
- Compound 17: N-((1,2,3,5,6,7-hexahydro-s-indacen-4-yl)carbamoyl)-1H-indole-6-sulfonamide;
- Compound 18: 5-fluoro-N-((1,2,3,5,6,7-hexahydro-s-indacen-4-yl)carbamoyl)-1H-indole-6-sulfonamide;
- Compound 19: N-((4-chloro-2,6-diisopropylphenyl)carbamoyl)-2-fluoro-5-(2-methyl-1,3-dioxolan-2-yl)benzenesulfonamide; and
- Compound 20: 2-fluoro-N-((1,2,3,5,6,7-hexahydro-s-indacen-4-yl)carbamoyl)-5-(2-methyl-1,3-dioxolan-2-yl)benzenesulfonamide.

6. Compound for use according to any one of claims 1, 2, 4, or 5, **characterized in that** the compound is N-((1,2,3,5,6,7-hexahydro-s-indacen-4-yl)carbamoyl)-4-(2-hydroxypropan-2-yl)furan-2-sulfonamide.

7. Compound for use as claimed in any of claims 1 to 6 in the treatment and/or prevention of atopic dermatitis.

8. Compound for use as claimed in any of claims 1 to 6 in the treatment and/or prevention of psoriasis.

9. Pharmaceutical composition comprising a compound as defined in any of claims 1 to 6 for use in the treatment and/or prevention of an inflammatory skin disorder, preferably atopic dermatitis or psoriasis.

10. Pharmaceutical composition for use according to claim 9, via topical application.
